# EUROPEAN PATENT APPLICATION

(11) **EP 4 339 957 A1**
(43) Date of publication of application: **20.03.2024**
(21) Application number: 22807184.1
(22) Date of filing: 18.03.2022
(51) Int. Cl.: G16B 45/00, C12P 21/08

(54) **ANTIBODY PRODUCTION ASSISTANCE METHOD AND ANTIBODY PRODUCTION ASSISTANCE PROGRAM**

(30) Priority: 13.05.2021 JP 2021081516
(71) Applicant: Shimadzu Corporation, Nakagyo-ku, Kyoto-shi, Kyoto 604-8511 (JP)
(72) Inventor: MOTOYAMA, Kento, Kyoto-shi, Kyoto 604-8511 (JP); YAMADA, Kaori, Kyoto-shi, Kyoto 604-8511 (JP); KANAI, Masaki, Kyoto-shi, Kyoto 604-8511 (JP); SUZUKI, Takashi, Kyoto-shi, Kyoto 604-8511 (JP)
(74) Representative: Kilian Kilian & Partner mbB
(86) International application number: PCT/JP2022/012559
(87) International publication number: WO 2022/239479

(57) **Abstract**

An antibody production assisting method for assisting production of an antibody includes: acquiring time-series data that indicates, in in chronological order, a plurality of metabolite data and a culture parameter, the plurality of metabolite data each corresponding to a respective amount of a plurality of metabolites generated by culturing a cell line for producing the antibody, the culture parameter being detected from the cell line in culture (Step S2); acquiring a degree of relevance between the culture parameter and each of the plurality of metabolites on the basis of the time-series data (Step S4); and generating display data based on the degree of relevance (Step S6).

## Description

### TECHNICAL FIELD

The present disclosure relates to an antibody production assisting method and an antibody production assisting program.

### BACKGROUND ART

In recent years, studies in research on antibodies produced by culturing cells have been advanced. The specification of U.S. Patent No. 8911964 (PTL 1) discloses a technique related to production of antibodies by using culture of Chinese hamster ovary (CHO) cells. PTL 1 discloses that manufacturers of antibodies monitor glucose concentrations and feedback the monitored results.

### CITATION LIST

### PATENT LITERATURE

PTL 1: Specification of U.S. Patent No. 8911964

### SUMMARY OF INVENTION

### TECHNICAL PROBLEM

The manufacturers of the antibodies preferably set appropriate culture conditions in the production of the antibodies. However, it is necessary for the manufacturers to set culture conditions on the basis of the limited monitored results. It has been a practice for the manufacturers to set the culture conditions on the basis of experience of the manufacturers themselves. Therefore, it may be difficult for some manufactures to set appropriate culture conditions, and in such a case, a problem may occur in that it is difficult to produce the antibodies.

An object of the present disclosure, which has been made to solve such a problem, is to assist production of antibodies.

### SOLUTION TO PROBLEM

An antibody production assisting method according to an aspect of the present disclosure is a method for assisting production of an antibody. The antibody production assisting method includes: acquiring time-series data that indicates, in in chronological order, a plurality of metabolite data and a culture parameter, the plurality of metabolite data each corresponding to a respective amount of a plurality of metabolites generated by culturing a cell line for producing the antibody, the culture parameter being detected from the cell line in culture. Also, the antibody production assisting method includes acquiring a degree of relevance between the culture parameter and each of the plurality of metabolites on the basis of the time-series data. Furthermore, the antibody production assisting method includes generating display data based on the degree of relevance.

Additionally, an antibody production assisting program according to an aspect of the present disclosure is a program for assisting production of an antibody. The antibody production assisting program causes a computer to execute acquiring of time-series data that indicates, in in chronological order, a plurality of metabolite data and a culture parameter, the plurality of metabolite data each corresponding to a respective amount of a plurality of metabolites generated by culturing a cell line for producing the antibody, the culture parameter being detected from the cell line in culture. Also, the antibody production assisting program causes the computer to execute acquiring of a degree of relevance between the culture parameter and each of the plurality of metabolites on the basis of the time-series data. Furthermore, the antibody production assisting program causes the computer to execute generating of display data based on the degree of relevance.

### ADVANTAGEOUS EFFECTS OF INVENTION

In the present disclosure, the display data based on the degree of relevance between the culture parameter and each of the plurality of metabolites is generated. Therefore, a manufacturer can presume a culture condition to be adjusted by viewing an image based on the display data. According to the present disclosure, it is thus possible to assist production of the antibody.

### BRIEF DESCRIPTION OF DRAWINGS

Fig. 1 is a configuration example of an assisting system 1 according to a present embodiment.
Fig. 2 is a diagram illustrating a hardware configuration example of an information processing device 100.
Fig. 3 is a diagram illustrating an example of transition of a culture parameter.
Fig. 4 is a diagram illustrating an example of transition of the culture parameter.
Fig. 5 is a diagram illustrating an example of transition of the culture parameter.
Fig. 6 is a diagram illustrating an example of transition of the amount of metabolites.
Fig. 7 is a diagram illustrating an example of transition of an oxygen partial pressure.
Fig. 8 is a functional block diagram of the information processing device and the like.
Fig. 9 is an example of a correspondence image.
Fig. 10 is a diagram illustrating an example of a metabolic map.
Fig. 11 is a flowchart illustrating main processing of the information processing device.
Fig. 12 is a diagram illustrating an example of a first image.
Fig. 13 is a diagram illustrating an example of a second image.
Fig. 14 is a diagram illustrating an example of a third image.
Fig. 15 is a diagram illustrating an example of a fourth image.
Fig. 16 is a diagram illustrating an example of transition of a culture parameter of a cell line A.
Fig. 17 is a diagram illustrating an example of transition of a culture parameter of a cell line B.
Fig. 18 is a diagram illustrating an example of transition of the amounts of metabolites of cell line A and cell line B.
Fig. 19 is a diagram illustrating an example of principal component score plots in principal component analysis.
Fig. 20 is a diagram illustrating an example of loading score plots in principal component analysis.
Fig. 21 is a diagram illustrating an example of a fifth image.
Fig. 22 is a diagram illustrating an example of the metabolic map.
Fig. 23 is a diagram illustrating an example of a sixth image.
Fig. 24 is an enlarged view of an image X of cell line A and cell line B.
Fig. 25 is a flowchart illustrating main processing of an information processing device according to another embodiment.
Fig. 26 is a diagram illustrating an example of a seventh image.
Fig. 27 is a diagram illustrating an example of an eighth image.

### DESCRIPTION OF EMBODIMENTS

Hereinafter, an embodiment of the present disclosure will be described in detail with reference to the drawings. Note that the same reference signs will be applied to the same or corresponding parts in the drawings and description thereof will not be repeated.

### [Configuration example of assisting system]

In the present embodiment, a manufacturer of an antibody (hereinafter, simply referred to as a "manufacturer" or a "user") sets an appropriate culture condition, a cultivation device cultures a cell line under the culture condition and extracts an antibody from the cultured cell line. In such a method for producing an antibody, it is preferable that an appropriate culture condition be set. The setting of the culture condition depends on a level of experience of the manufacturer himself/herself in many cases, and it may be difficult for a less experienced manufacturer to set an appropriate culture condition. Thus, an antibody production assisting method according to the present embodiment assists production of an antibody to be used in an antibody drug. More specifically, the antibody production assisting method allows the manufacturer to predict a culture condition to be set and a value of the culture condition.

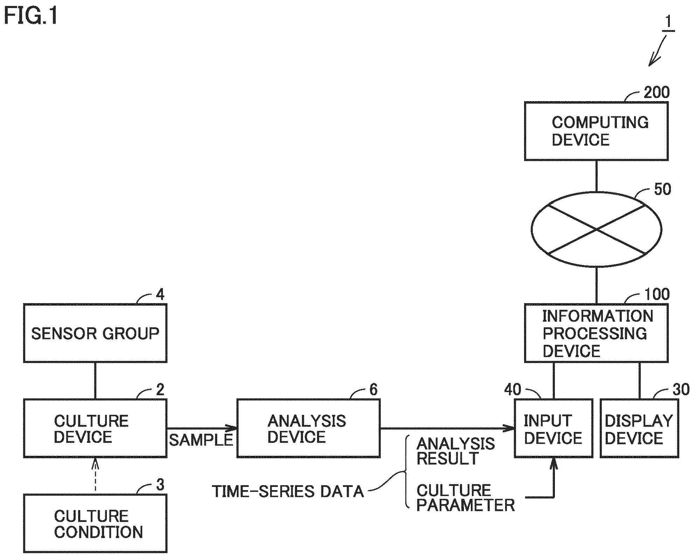

Fig. 1 is a configuration example of an assisting system 1 according to the present embodiment. Assisting system 1 includes a culture device 2, a sensor group 4, an analysis device 6, an information processing device 100, a computing device 200, a display device 30, and an input device 40. Display device 30 and input device 40 are connected to information processing device 100.

Culture device 2 cultures a cell line and extracts an antibody from the cultured cell line. The cell line is, for example, a CHO cell. Examples of a culturing method include alternating tangential flow (ATF) culture as a kind of perfusion culturing method, fed-batch culture as a kind of feeding culturing method, and the like. Particularly, since a perfusion system is employed in the ATF culture, it is possible for a manufacturer to achieve continuous culture, that is, to culture a cell line in a long-term culture period. In the present embodiment, culture device 2 employs the ATF culturing method. It is possible to achieve long-term cell culture by the ATF culturing method being employed. In the present embodiment, it is assumed that the cell line is cultured for a 30-day culture period.

Also, a culture condition 3 that the manufacturer can set is, for example, at least one of an amount of basal medium, a composition of a basal medium, a flow rate of breeding, an amount of feed medium, a composition of a feed medium, a perfusion rate, a stirring speed, an amount of air blowing, an amount of carbon dioxide blowing, an amount of alkali addition, an amount of defoaming agent addition, and the like. The manufacturer sets at least one of these culture conditions as a culture condition through an operation or the like of a device corresponding to the culture condition. For the amount of carbon dioxide blowing, for example, the manufacturer sets the amount of carbon dioxide blowing by operating a carbon dioxide blowing device.

Sensor group 4 is composed of one or more sensors. Each of the one or more sensors detects a culture parameter corresponding to the sensor. The culture parameter includes a cell density, a cell viability, a cell diameter, a specific growth speed, an antibody production speed, pH, a temperature, dissolved oxygen (DO), an oxygen partial pressure, a carbon dioxide partial pressure, or the like. The specific growth speed is a proportion of an amount of increase in cells per unit time calculated from a difference from a previous (one day before, for example) cell density, or the like.

The manufacturer extracts a part of culture solution for the cell line cultured in culture device 2. Then, the manufacturer causes analysis device 6 to analyze the extracted part of cell culture solution as a sample. At that time, analysis device 6 performs an operation for separating a culture solution supernatant and cells, a pre-analysis treatment is performed on each of them, and analysis device 6 is then caused to perform analysis. Analysis device 6 is, for example, a liquid chromatograph-mass spectrometry/mass spectrometry (LC-MS/MS). Also, the manufacturer also inputs the date and the time of the analysis to analysis device 6.

Analysis device 6 can measure the amount of each of metabolites (171 metabolites, for example) generated by culturing a cell line. The manufacturer can select the number of metabolites to be measured and the types of metabolites. Then, analysis device 6 derives an analysis result (the amount of each of the plurality of metabolites) in an associated manner with the input date and time. The analysis result includes a plurality of (171, for example) metabolite data that indicate, in in chronological order, the amount of each of the plurality of metabolites generated by culturing the cell line. The metabolite data may be data that directly indicates the amount of each of the metabolites, or may be data that indirectly indicates it. In other words, the plurality of metabolite data are data corresponding to the amount of each of the plurality of metabolites. In the present embodiment, the manufacturer causes analysis device 6 to analyze the sample once a day and derive an analysis result. Also, the manufacturer may cause a display device (not illustrated) of analysis device 6 to display the analysis result or may not cause the display device to display it every time the analysis result is derived. In this manner, the manufacturer causes analysis device 6 to derive the analysis results for the culture period (that is, for 30 days).

Also, the manufacturer acquires a culture parameter in the culture period on the basis of a detection result of sensor group 4. For example, the manufacturer acquires the culture parameter when the cells cultured by culture device 2 are collected.

Input device 40 is a device such as a keyboard or a mouse, for example, and receives a command from the manufacturer. Display device 30 is composed of a liquid crystal display (LCD) panel, for example, and displays information for the manufacturer. In a case where a touch panel is used as a user interface, input device 40 and display device 30 are integrally formed.

Information processing device 100 generates display data by executing predetermined processing on data transmitted from input device 40. Information processing device 100 may be implemented by a general-purpose computer or may be implemented by a computer dedicated for information processing device 100, for example. The general-purpose computer may be a personal computer (PC) or may be a tablet, for example. Note that at least a part of computing for generating the display data may be implemented as software by a central processing unit (CPU) of information processing device 100 or may be implemented as hardware that performs processing independently from the CPU.

The manufacturer inputs the analysis results in the culture period and the culture parameters to input device 40. In the present embodiment, the manufacturer inputs the analysis results for 30 days and the culture parameters for 30 days corresponding to the analysis results to input device 40. Note that not all the analysis results for 30 days are necessarily be input, and analysis results for predetermined days out of the 30 days (analysis results for 15 days, for example), for example, may be input. Information processing device 100 acquires the data input from input device 40 as time-series data for 30 days. In the present embodiment, the time-series data refers to the analysis results for 30 days and each culture parameter for the 30 days.

Information processing device 100 and computing device 200 are configured to be able to communicate via a network 50. Once the time-series data is acquired from input device 40, then information processing device 100 outputs it to computing device 200. Computing device 200 executes predetermined computing, which will be described later, and outputs the computing result to information processing device 100. Information processing device 100 generates display data based on the computing result.

### [Hardware configuration of information processing device]

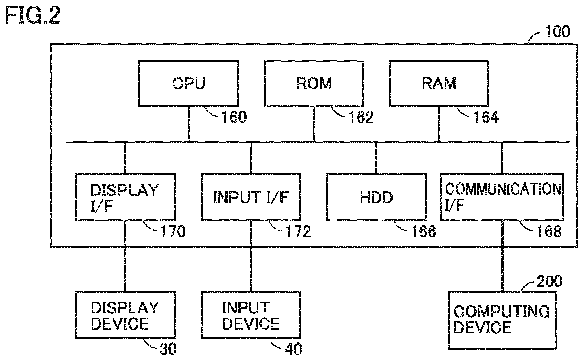

Fig. 2 is a diagram illustrating a hardware configuration example of information processing device 100. Referring to Fig. 2, information processing device 100 has, as main components, a CPU 160, a read only memory (ROM) 162, a random access memory (RAM) 164, a hard disk drive (HDD) 166, a communication interface (I/F) 168, a display I/F 170, and an input I/F 172. The components are connected to each other via a data bus.

Communication I/F 168 is an interface for communicating with computing device 200. Display I/F 170 is an interface for communicating with display device 30. Input I/F 172 is an interface for communicating with input device 40.

ROM 162 stores a program to be executed by CPU 160. RAM 164 can temporarily store data generated by CPU 160 executing the program and data input via communication I/F 168. RAM 164 can function as a transitory data memory used as a work area. HDD 166 is a non-volatile storage device. A semiconductor storage device such as a flash memory may be employed instead of HDD 166.

The program stored in ROM 162 may be stored in a non-transitory recording medium and may be distributed as a program product. Alternatively, the program may be provided by an information provider as a product program that can be downloaded through what is called the Internet or the like. Information processing device 100 reads the program provided by the recording medium, the Internet, or the like. Information processing device 100 stores the read program in a predetermined storage region (in ROM 162, for example). CPU 160 can execute various kinds of processing by executing the program.

### [Concerning culture parameters]

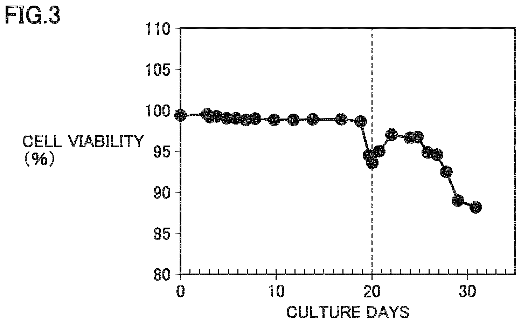

Next, culture parameters and the like will be described. Figs. 3 to 5 are graphs showing culture parameters for 30 days in in chronological order.

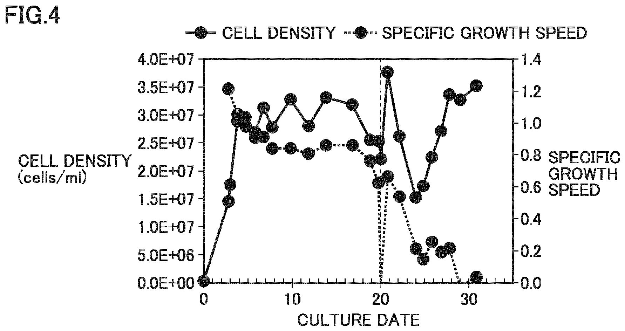

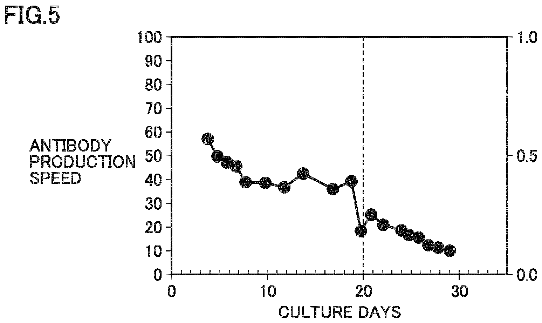

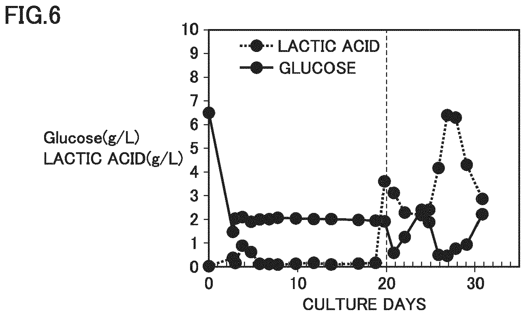

In Figs. 3 to 6, the horizontal axis represents the number of culture days. Also, the vertical axis in Fig. 3 represents the cell viability as a culture parameter. The vertical axis in Fig. 4 represents a cell density and a specific growth speed as culture parameters. Fig. 5 illustrates an antibody production speed as a culture parameter. Fig. 6 illustrates the amounts of lactic acid and glucose as generated metabolites. The graphs in Figs. 3 to 6 are displayed on a predetermined display device (display device 30, for example), such that the manufacturer can view these graphs.

In the examples in Figs. 3 to 6, the cell viability, the cell density, the specific growth speed, and the antibody production speed significantly vary on nineteenth and twentieth days of the culture days as illustrated by the dashed lines. Moreover, in the example in Fig. 6, the amounts of glucose and lactic acid significantly vary on the nineteenth and twentieth days of the culture days. Therefore, the manufacturer can presume abnormality of the culture parameters on the nineteenth and twentieth days of the culture days.

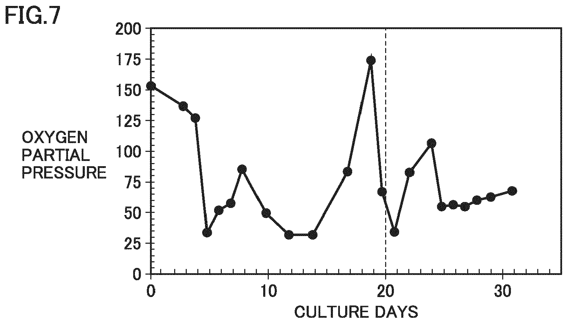

Under this presumption, it is assumed that the manufacturer has also checked an oxygen partial pressure as a culture parameter. Fig. 7 is a graph illustrating the oxygen partial pressure for 30 days in in chronological order. As illustrated by the dashed line in Fig. 7, the oxygen partial pressure also significantly varies on the nineteenth and twentieth days of the culture days.

In such a process of manufacturing the antibody, the cultured cell line is likely to be affected by a change in environment inside a culture tank of a first culture device 10 and the like, and variations in metabolites frequently occur. Thus, it is preferable for the manufacturer to set (adjust) the aforementioned culture conditions such that the variations in the culture parameters illustrated in Figs. 3 to 6 and the amount of metabolite are stabilized. However, it is necessary for the manufacturer to set the culture conditions on the basis of limited monitor results (for example, the monitor results in Figs. 3 to 7). In the related art, manufacturers set culture conditions on the basis of experience of the manufacturers themselves. Therefore, it may be difficult for some manufactures to set appropriate culture conditions, and in such a case, a problem that it is difficult to produce antibodies may occur.

Incidentally, metabolites (glucose, a lactic acid, and the like in Fig. 6) are typically produced in the process of manufacturing antibodies. Also, it is technically difficult to artificially control the production of the metabolites. The inventor of the present disclosure focused on degrees of relevance between the culture parameters and the metabolites. Then, information processing device 100 displays an image based on the degrees of relevance. This image will be described later. It is thus possible for the manufacturer to presume culture conditions to be adjusted on the basis of the image. Accordingly, it is possible to assist production of the antibodies in the present embodiment.

### [Functional block diagram of information processing device]

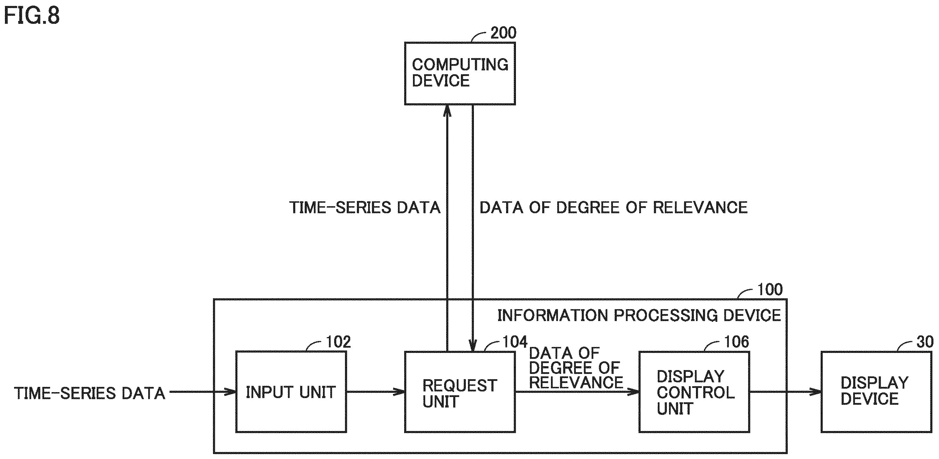

Fig. 8 is a functional block diagram of information processing device 100 and the like. Information processing device 100 has an input unit 102, a request unit 104, and a display control unit 106. The aforementioned time-series data is input to input unit 102. As the time-series data, comma separated value (CSV) data, for example, is input to input unit 102. The manufacturer may manually input the time-series data to input unit 102. Also, analysis device 6 may input the time-series data to input unit 102. The time-series data is output to request unit 104. Request unit 104 outputs a request signal and the time-series data to computing device 200. The request signal is a signal for requesting data of degrees of relevance, which will be described later, from computing device 200.

Computing device 200 receives the request signal and the time-series data. Computing device 200 executes correlation analysis on the time-series data. Although a method using a Pearson product-moment correlation coefficient or a Spearman's or Kendall's rank correlation coefficient is used as a method for the correlation analysis in accordance with properties of analysis data, a method other than these may also be used as a method for analyzing a correlation between two different values. Computing device 200 generates data of degrees of relevance through the correlation analysis. The data of degrees of relevance is data indicating degrees of relevance between the culture parameters and each of the plurality of metabolites. Computing device 200 transmits the data of degrees of relevance to information processing device 100.

Request unit 104 of information processing device 100 acquires the data of degrees of relevance. Request unit 104 transmits the data of degrees of relevance to display control unit 106. Display control unit 106 generates display data based on the data of degrees of relevance. Display control unit 106 causes display device 30 to display an image based on the display data. Note that information processing device 100 rather than computing device 200 may execute the correlation analysis.

### [Correspondence image]

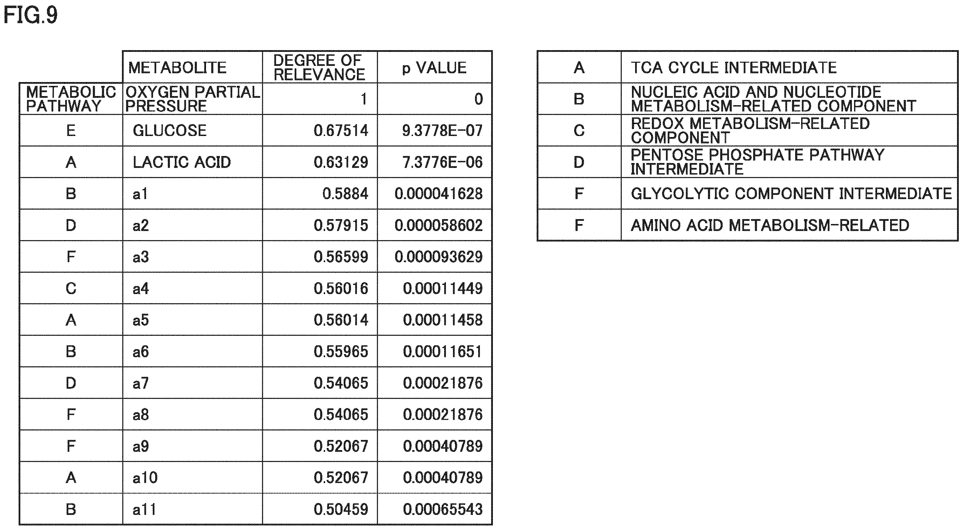

Fig. 9 is an example of an image (correspondence image) displayed on display device 30. As illustrated in the left table in Fig. 9, the correspondence image is illustrated in the table format (matrix format). In the example in Fig. 9, a section for a metabolic pathway, a section for a name of a metabolite, a section for a degree of relevance, and a section for a p value are represented in an associated manner. Moreover, a name of a target culture parameter is shown in a block below a block for a name of each section (a metabolic pathway, a metabolite, a degree of relevance, and a p value). In the example in Fig. 9, the culture parameter is an "oxygen partial pressure".

Also, identification information A to E for the metabolic pathway of the metabolite corresponding to the section for the metabolic pathway is shown in the section for the metabolic pathway. Additionally, a degree of relevance between the metabolite and the culture parameter (oxygen partial pressure) corresponding to the section for the degree of relevance is shown in the section for the degree of relevance. In addition, a p value for the degree of relevance corresponding to the section for the p value is shown in the section for the "p value".

In the right table in Fig. 9, what metabolic pathway each identification information A to E indicates is displayed. For example, the identification information A indicates a metabolic pathway for a TCA cycle intermediate in the example in Fig. 9. Also, the identification information B indicates a metabolic pathway for a urea cycle. Also, the identification information C indicates a metabolic pathway for glutathione metabolism. Also, the identification information D indicates a metabolic pathway for a pentose phosphate pathway intermediate. Also, the identification information E indicates a metabolic pathway for a glycolytic component intermediate.

Additionally, the left table in Fig. 9 shows the metabolic pathway identification information E (glycolytic component intermediate), a degree of relevance of 0.67514, and a p value of 9.3778E-07 for glucose as a metabolite, for example. Also, it shows the metabolic pathway identification information A (TCA cycle intermediate), a degree of relevance of 0.63129, and a p value of 7.3776E-06 for a lactic acid as a metabolite. As other metabolites, metabolites a1 to a11 are displayed, and a metabolic pathway, a degree of relevance, and a p value of each of the metabolites a1 to a11 are shown. In this manner, information processing device 100 may display names of the metabolites in a different manner (in association with different identification information) for each of metabolic pathways of the metabolites. Also, information processing device 100 may display the names of the metabolites with a different color for each of the metabolic pathways of the metabolites in a modification example.

Also, analysis device 6 measures the amount of each of the 171 kinds of metabolites as described above. Thus, display device 30 may display a degree of relevance and the like of each of the 171 kinds of metabolites. However, display of metabolites with low degrees of relevance with the culture parameters are not very useful for the manufacturer. Also, it becomes difficult for the manufacturer to see the correspondence image if degrees of relevance are displayed for a large number of metabolites.

Thus, information processing device 100 extracts degrees of relevance that satisfy a condition for a degree of relevance indicating that a degree of relevance with a culture parameter is high in the present embodiment. Here, the condition for a degree of relevance in the present embodiment is a condition that the p value is less than 0.05 and an absolute value of the degree of relevance is more than 0.7.

In this manner, the correspondence image in Fig. 9 is an image in which each of the names of the plurality of metabolites (glucose, a lactic acid, a1 to a11) is associated with a degree of relevance between the culture parameter (oxygen partial pressure) and each of the plurality of metabolites for the culture parameter. Furthermore, the correspondence image in Fig. 9 is an image in which the degrees of relevance that satisfy the aforementioned condition for a degree of relevance are associated with the names of metabolites associated with the degrees of relevance.

Incidentally, the manufacturer can cause information processing device 100 to create a metabolic map. The metabolic map is a map that shows metabolites, metabolic pathways of the metabolites, and the like. Also, an application for assisting creation of the metabolic map has been downloaded on information processing device 100, and the manufacturer creates the metabolic map by using the application for assisting creation.

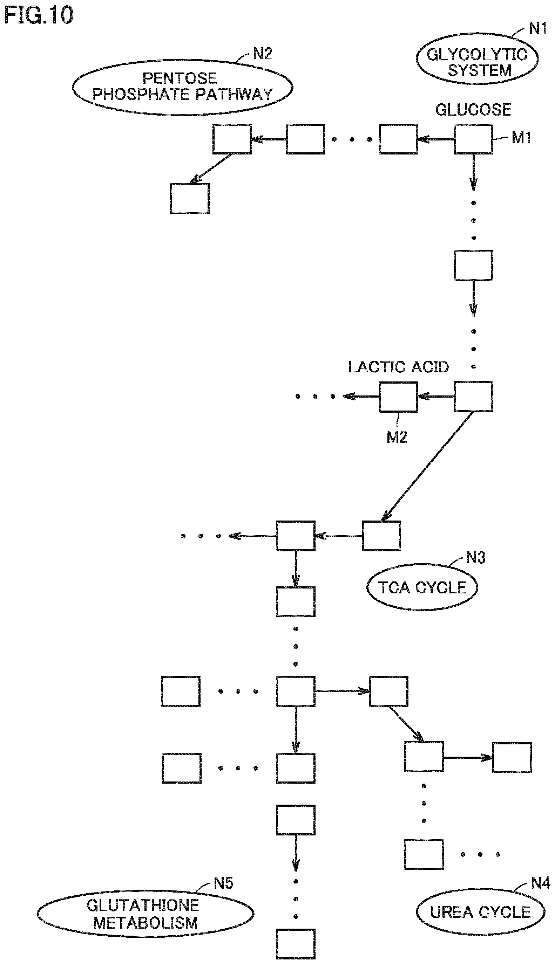

Fig. 10 is a diagram illustrating an example of the metabolic map. In the example in Fig. 10, an example of the metabolic map is omitted. In the example in Fig. 10, metabolites to be generated, metabolic pathways, and the like are shown. In the example in Fig. 10, identification information N1 for a metabolic pathway of a glycolytic system, identification information N2 for a metabolic pathway of a pentose phosphate pathway, identification information N3 for a metabolic pathway of a TCA cycle, identification information N4 for a metabolic pathway of a urea cycle, and identification information N5 for a metabolic pathway of a glutathione cycle are displayed as identification information for metabolic pathways.

Also, glucose and a lactic acid are shown as metabolites, and names of other metabolites are omitted in the example in Fig. 10. Additionally, related information related to the metabolites is displayed below the names of the metabolites. The related information is, for example, a graph showing a culture parameter (for example, a specific growth speed) of the metabolite in the culture period (30 days). For example, related information M1 of glucose is a graph showing a specific growth speed of glucose in the culture period. Also, related information M2 of the lactic acid is a graph showing a specific growth speed of the lactic acid in the culture period. In addition, the manufacturer may switch the related information (specific growth speed) to other related information (for example, cell viability) by executing a predetermined switching operation on input device 40.

Also, the manufacturer can estimate a culture condition to be adjusted by viewing the correspondence image in Fig. 9 and the metabolic map in Fig. 10. For example, the manufacturer can estimate the culture condition to be adjusted by viewing each metabolite or the like included in the metabolic map around metabolites with high degrees of relevance from the entire metabolic map. Furthermore, the manufacturer can also estimate how much the culture condition to be adjusted is to be adjusted by viewing the metabolic map in the surroundings. Note that the metabolic map in Fig. 10 may display the culture parameters of the metabolite in an associated manner with each metabolite.

### [Flowchart of information processing device]

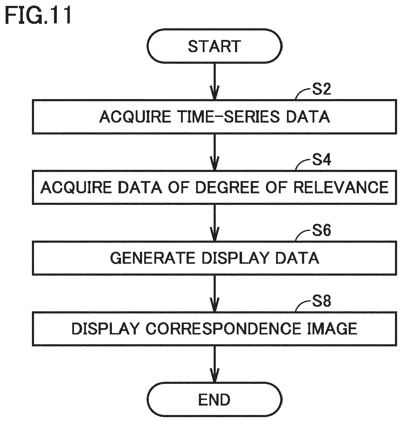

Fig. 11 is a flowchart illustrating main processing of information processing device 100. In the example in Fig. 11, information processing device 100 acquires time-series data in Step S2 first. Next, information processing device 100 outputs a request signal and the time-series data to computing device 200 in Step S4. Computing device 200 executes correlation analysis on the time-series data. Computing device 200 generates data of degrees of relevance through the correlation analysis. Computing device 200 transmits the data of degrees of relevance to information processing device 100, and information processing device 100 thereby acquires the data of degrees of relevance.

Next, information processing device 100 generates display data on the basis of the data of degrees of relevance in Step S6. Then, information processing device 100 displays a correspondence image based on the display data on display device 30 in Step S8.

### [Other display images]

Next, other display images of the correspondence image displayed by display device 30 will be described. For example, an image (first image) of only a metabolic map in the surrounding of a metabolite may be displayed while metabolic map at the other location may not be displayed once the manufacturer designates a block of a name of the metabolite from among names of the plurality of metabolites displayed on the correspondence image in Fig. 9.

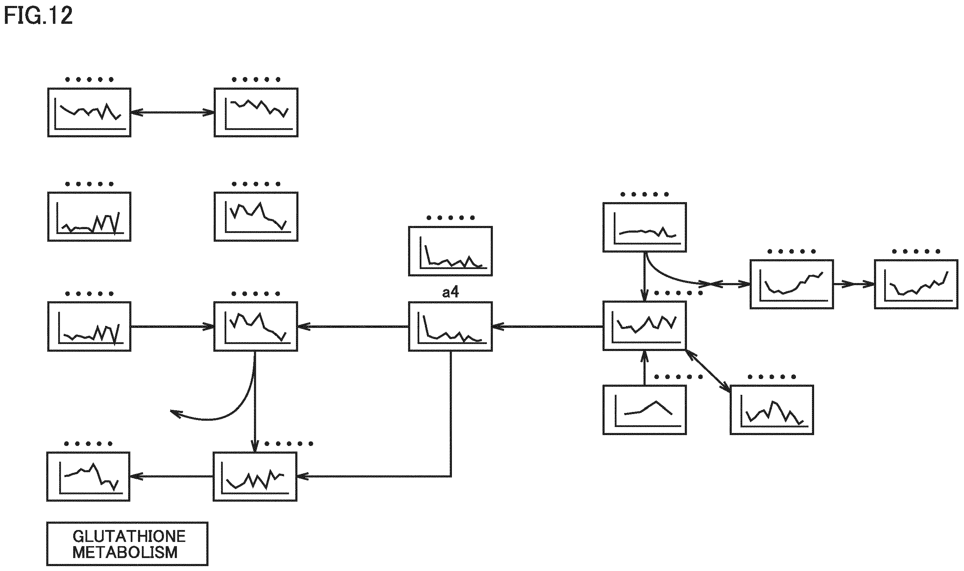

Fig. 12 is an example of the image (first image) of the metabolic map around the designated metabolite. The example in Fig. 12 is a metabolic map displayed in a case where metabolite a4 is designated from among the plurality of metabolites in Fig. 9. In the example in Fig. 9, metabolite a4 is a glutathione metabolic pathway, and a metabolic map corresponding to the glutathione metabolic pathway in the metabolic map is displayed. In this manner, only the metabolic map around the designated metabolite from among the plurality of metabolites corresponding to the degrees of relevance that satisfy the condition for a degree of relevance is displayed while the metabolic map at the other location is not displayed in the example in Fig. 12. With such a configuration, the manufacturer can more clearly identify the metabolic map around the designated metabolite. Therefore, the manufacturer can more clearly presume the culture condition to be adjusted on the basis of the metabolic map around the designated metabolite.

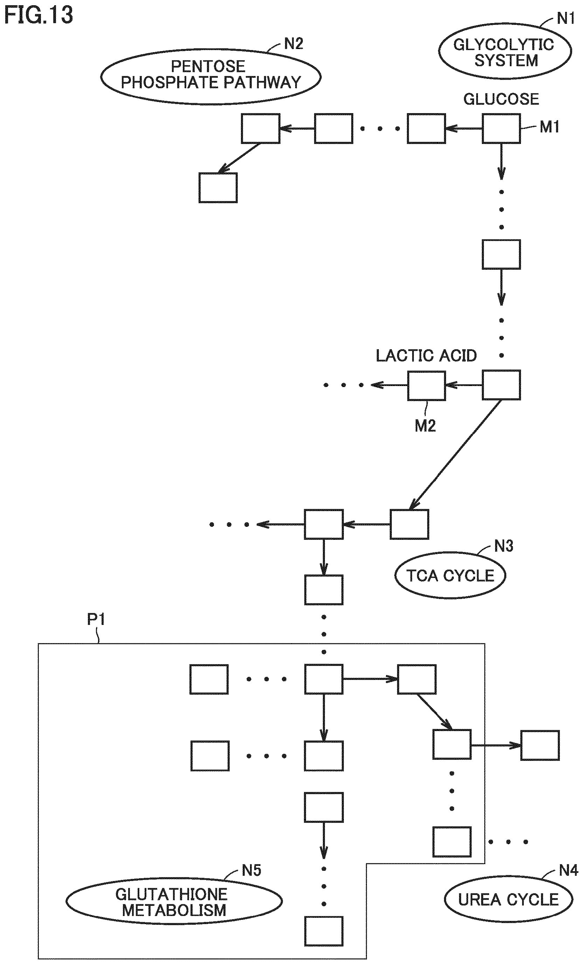

Also, information processing device 100 may display the metabolic map displayed in a case where designation is made from among the plurality of metabolites as an image (second image) in a different manner from the metabolic map at the other location. Fig. 13 is a diagram illustrating an example of the second image. In the example in Fig. 13, the glutathione metabolic pathway is displayed by being surrounded by a frame P1. In other words, the metabolic map of the glutathione metabolic pathway is displayed in a different manner from the metabolic map at the other location. With such a configuration, the manufacturer can more clearly identify the metabolic map around the designated metabolite. Therefore, the manufacturer can more clearly presume the culture condition to be adjusted on the basis of the metabolic map around the designated metabolite. Note that the different manner is not limited to that in the example in Fig. 13 and another manner may be used. For example, information processing device 100 may blink the metabolic map displayed in a case where designation is made from among the plurality of metabolites.

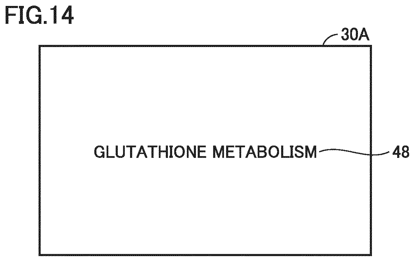

Also, information processing device 100 may display a third image including a name of a metabolic pathway of the designated metabolite. Fig. 14 is an example of the third image. As illustrated in Fig. 14, a name image 48 of the metabolic pathway is displayed in a display region 30A on display device 30. In the example in Fig. 14, name image 48 is a letter image of "glutathione metabolism". With such a configuration, the manufacturer can recognize the metabolic pathway of the metabolite with a high degree of relevance with the culture parameter. Therefore, the manufacturer can more clearly presume the culture condition to be adjusted on the basis of the metabolic pathway. Note that display region 30A is not described in Figs. 12 and 13 and Fig. 15, which will be described later, or the like.

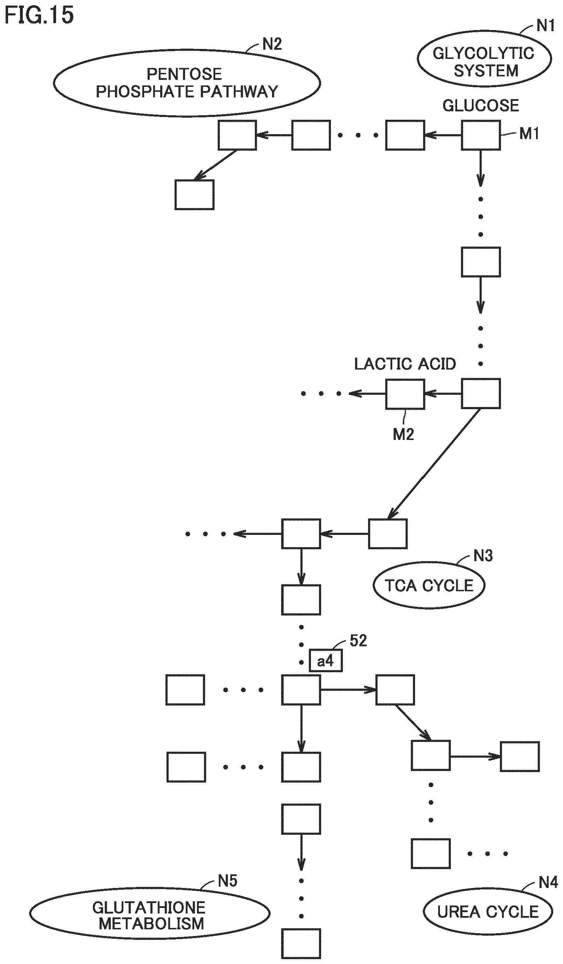

Also, information processing device 100 may display a fourth image such that the designated metabolite is identifiable in the metabolic map. Fig. 15 is an example of the fourth image. As illustrated in Fig. 15, information processing device 100 displays the metabolic map. Along with this, information processing device 100 displays the designated metabolite in a different manner from the other metabolites in the metabolic map. In the example in Fig. 15, it is assumed that the metabolite a4 has been designated by the manufacturer. In this case, information processing device 100 displays the designated metabolite a4 in a different manner from the other metabolites. In the example in Fig. 15, the name of the metabolite a4 is displayed in a manner in which the name is surrounded by a frame 52. With such a configuration, the manufacturer can recognize the metabolite with a high degree of relevance with the culture parameter. Therefore, the manufacturer can more clearly presume the culture condition to be adjusted on the basis of the metabolite. Note that the different manner is not limited to that in the example in Fig. 15 and another manner may be used. For example, information processing device 100 may perform blinking or the like of the designated metabolite.

Also, the case where there are multiple metabolites (thirteen in the example in Fig. 9) with high degrees of relevance with the culture parameter has been described in the present embodiment. However, in a case where there is one metabolite with a high degree of relevance with the culture parameter, any of the first image to the fourth image may be displayed for the one metabolite.

### [Time-series data of multiple attributes]

Next, time-series data of multiple attributes will be described. Among cells with complicated gene sequences, such as a CHO cell line that has acquired an indefinite proliferative potential, some created cells may not have intended gene sequences. Therefore, significant differences may occur in culture results even if a plurality of cell lines are cultured under mutually the same culture conditions. Hereinafter, two cell lines will be referred to as a cell line A and a cell line B. In the antibody production assisting method according to the present embodiment, the manufacturer is allowed to presume a culture condition to be adjusted in culture device 2 such that an antibody produced from cell line A and an antibody produced from cell line B are appropriately produced. In the present embodiment, the plurality of first metabolite data that indicate, in chronological order, each of amounts of a plurality of metabolites generated by culturing cell line A and the plurality of second metabolite data that indicate, in chronological order, each of amounts of a plurality of metabolites generated by culturing cell line B are used. Then, information processing device 100 specifies a target metabolite caused by a difference between the first metabolite data and the second metabolite data and displays an image based on the target metabolite.

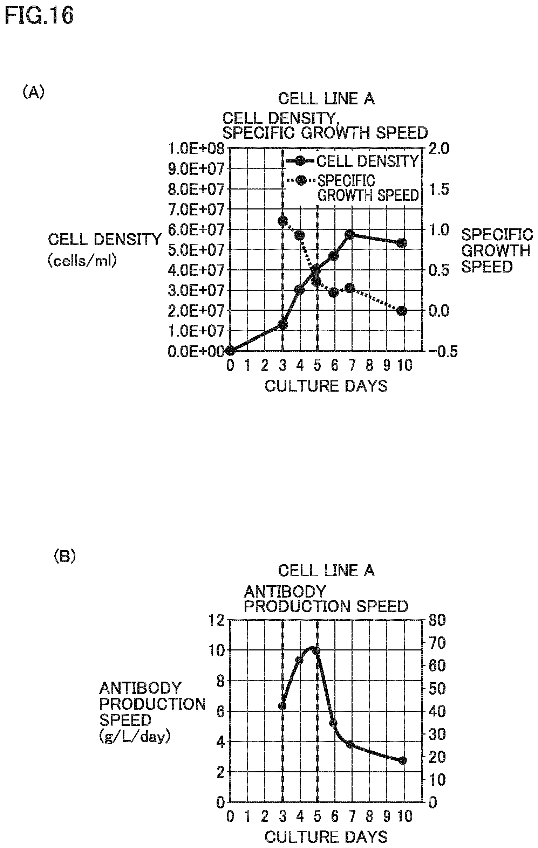

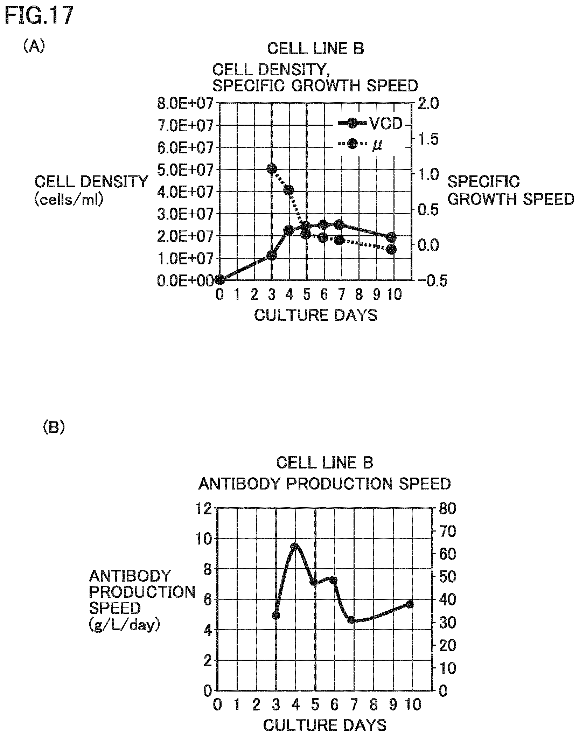

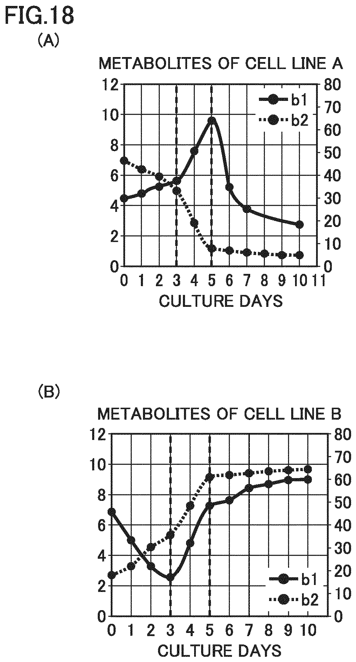

Fig. 16 is a graph illustrating, in in chronological order, culture parameters of cell line A for ten days. Fig. 17 is a graph illustrating, in in chronological order, culture parameters of cell line B for ten days. Fig. 18 is a graph illustrating, in in chronological order, a plurality of metabolites generated by culturing the cell lines for ten days. In the example in Fig. 18, a metabolite b1 and a metabolite b2 are illustrated as the plurality of metabolites.

In Figs. 16 to 18, the horizontal axis represents the number of culture days. Also, the vertical axis in Fig. 16(A) represents a cell density and a specific growth speed as culture parameters of cell line A. The vertical axis in Fig. 16(B) represents an antibody production speed as a culture parameter of cell line A. The vertical axis in Fig. 17(A) represents a cell density and a specific growth speed as culture parameters of cell line B. The vertical axis in Fig. 17(B) represents an antibody production speed as a culture parameter of cell line B. The graphs in Figs. 16 to 18 are displayed on a predetermined display device (for example, display device 30), and the manufacturer can view these graphs.

In the example in Figs. 16 and 17, the cell densities, the specific growth speeds, and the antibody production speeds of cell line A and cell line B significantly vary on the third to fifth days in the culture days as illustrated by the dashed lines. Furthermore, in the example in Fig. 18, the amounts of metabolite b1 and metabolite b2 significantly vary on the third to fifth days in the culture days. Therefore, the manufacturer can presume abnormality of the culture parameters on the third to fifth days in the culture days. Note that the period from the third day to the fifth day is typically a period from a cell growth period to a stationary period (a period during which convergence of the cell growth and acceleration of antibody production occur).

In the process of manufacturing the antibody, cell line A and cell line B to be cultured are likely to be affected by a change in environment inside the culture tank of culture device 2 and the like, and variations in metabolites frequently occur. Thus, it is preferable that the manufacturer set (adjust) the culture condition in culture device 2 such that cell line A and cell line B have equivalent trends of variations in amounts of metabolites. However, it is necessary for the manufacturer to set the culture condition in culture device 2 on the basis of the limited monitored results (the monitored results in Figs. 16 to 18, for example). In the related art, manufactures set culture conditions in culture device 2 on the basis of experience of the manufacturers themselves. Therefore, it may be difficult for some manufactures to set appropriate culture conditions, and in such a case, a problem that it is difficult to produce antibodies in culture device 2 may occur.

Incidentally, the metabolites (metabolite b 1, metabolite b5, and the like in Fig. 18) are typically produced in the process of manufacturing the antibody. Also, it is technically difficult to artificially control the production of the metabolites. The inventor of the present disclosure focused on a difference between the trend of variations in amount of metabolite of cell line A and the trend of variations in amount of metabolite of cell line B. Then, information processing device 100 specifies a target metabolite caused by the difference and displays an image corresponding to the target metabolite. The image will be described later. Therefore, the manufacturer can presume the culture condition to be adjusted in culture device 2 on the basis of the image such that the antibody produced from cell line A and the antibody produced from cell line B are appropriately produced. Therefore, it is possible to assist production of the antibodies in the present embodiment.

In this embodiment, the aforementioned first metabolite data and second metabolite data are input to input unit 102 illustrated in Fig. 8. The manufacturer may manually input the first metabolite data and the second metabolite data to input unit 102. Also, analysis device 6 may input the first metabolite data and the second metabolite data to input unit 102. The first metabolite data and the second metabolite data are output to request unit 104. Request unit 104 outputs a request signal and the metabolite data to computing device 200. The request signal is a signal for requesting focused metabolite data, which will be described later, from computing device 200.

Computing device 200 receives the request signal, the first metabolite data, and the second metabolite data. Computing device 200 executes principal component analysis on the first metabolite data and the second metabolite data. Computing device 200 generates focused metabolite data through the principal component analysis. The focused metabolite data is data indicating a target metabolite or the like that is attributable to a difference between the first metabolite data and the second metabolite data. Computing device 200 transmits the focused metabolite data to information processing device 100.

Request unit 104 of information processing device 100 acquires the focused metabolite data. Request unit 104 transmit the focused metabolite data to display control unit 106. Display control unit 106 generates display data based on the focused metabolite data. Display control unit 106 causes display device 30 to display an image based on the display data.

As described in Figs. 16 to 18, three culture parameters (cell densities, specific growth speeds, and antibody production speeds) of both cell line A and cell line B vary in the period from the third day to the fifth day in the culture period. Thus, samples of the three days (three samples) of each of cell line A and cell line B will be reviewed. The manufacturer inputs metabolite data (data indicating the amounts of 171 metabolites) of each of eighteen groups (= 2 × 2 × 3) to input device 40 (see Fig. 1). The metabolite data of the eighteen groups is transmitted to computing device 200, and computing device 200 executes multivariable analysis (for example, principal component analysis) on the metabolite data of the eighteen groups.

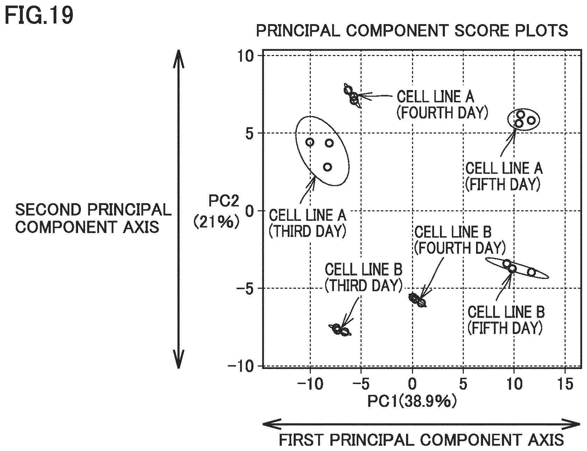

Fig. 19 is an example of principal component score plots by principal component analysis. The horizontal axis in Fig. 19 is a first principal component axis. Additionally, the vertical axis in Fig. 19 is a second principal component axis. The midpoint of the first principal component axis is "0", and the midpoint of the second principal component axis is "0".

In the example in Fig. 19, the three plot of cell line A (third day), the three plot of cell line A (fourth day), the three plot of cell line A (fifth day), the three plot of cell line B (third day), the three plot of cell line B (fourth day), and the three plot of cell line B (fifth day) are illustrated.

In the example in Fig. 19, the plots of cell line A and cell line B on the third day are distributed in a negative region of the first principal component axis. Also, the plots of cell line A and cell line B on the fifth day are distributed in a positive region of the first principal component axis. Therefore, the first principal component axis is an axis that can explain the time axis (the number of culture days).

Also, plots of cell line A are distributed in a positive region of the second principal component axis. Additionally, plots of cell line B are distributed in a negative region of the second principal component axis. Therefore, the second principal component axis is an axis that can explain the culture parameter (or the culture condition).

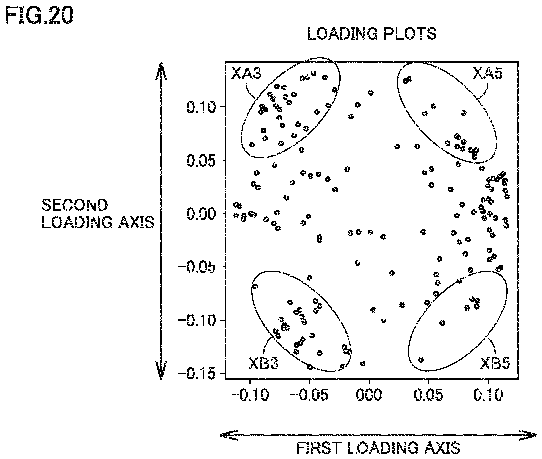

Fig. 20 is an example of loading score plots by principal component analysis. The plurality of plots illustrated in Fig. 20 are plots, each of which indicates each of the plurality of metabolites. In practice, a name of the metabolite is displayed in association with each of the plurality of plots in Fig. 20, the name of the metabolite is omitted in the example in Fig. 20.

In the example in Fig. 20, the horizontal axis is a first loading axis and corresponds to the first principal component axis in Fig. 20. Therefore, the first loading axis is an axis that can explain a time axis. Also, the vertical axis in the example in Fig. 20 is a second loading axis and corresponds to the second principal component axis in Fig. 20. Therefore, the second loading axis is an axis that can explain a culture parameter.

In the example in Fig. 20, metabolite plots indicating large values (values whose absolute values are large) in a negative region of the first loading axis correspond to cell line A (third day) and are thus surrounded by a frame XA3. Also, metabolite plots indicating large values in a positive region of the first loading axis correspond to cell line A (fifth day) and are thus surrounded by a frame XA5. Metabolite plots indicating large values (values whose absolute values are large) in a negative region of the second loading axis correspond to cell line B (third day) and are thus surrounded by a frame XB3. Metabolite plots indicating large values in a positive region of the second loading axis correspond to cell line B (fifth day) and are thus surrounded by a frame XB5.

It is only necessary for the manufacture to view the names of the metabolites inside frame XA3 in Fig. 20 in a case where it is desired to recognize the metabolite of cell line A with a trend of a large value on the third day, for example. Also, the plurality of metabolite plots are present inside frame XA3. Information processing device 100 calculates the amount of PC 1 principal component load and the amount of PC2 principal component load from a loading value (PC1 column) corresponding to a first principal component score and a loading value (PC2 column) corresponding to a second principal component score, for example. These values are correlation coefficients between the principal scores and each metabolite substance level, and it is thus possible to interpret an absolute value of the amount of principal component load of 0.4 or more as a component showing a slightly strong correlation and an absolute value of 0.7 or more as a component showing a strong correlation in accordance with a typical definition of the correlation coefficient. In this manner, a target metabolite that shows a strong correlation with a categorization pattern of a data group indicated by the principal component score plots is specified. The target metabolite is a metabolite that is attributable to a difference between the amount of metabolite of cell line A and the amount of metabolite of cell line B. Additionally, the specification of the target metabolite may be executed on the basis of a p value by calculating the p value by using the amount of principal component load.

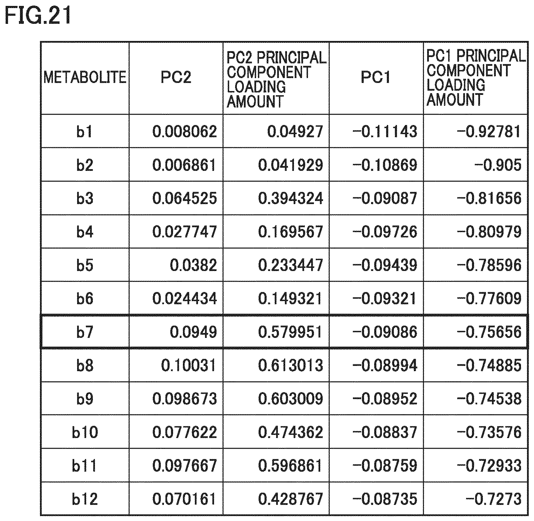

Fig. 21 is an example of an extraction result for the third day of cell line A. Information processing device 100 causes display device 30 to display an image of the extraction result. Information processing device 100 may cause display device 30 to display the image (fifth image) of the extraction result. The image of the extraction result corresponds to the "image based on the target metabolite" in the present disclosure. In the example in Fig. 21, plurality of metabolites b1 to b 12 of plots surrounded by a frame XA3 are shown. Plurality of metabolites b1 to b 12 are candidate metabolites of the target metabolite. Furthermore, information processing device 100 specifies target metabolite b7 that is most attributable to the above difference from among candidate metabolites b 1 to b 12. Information processing device 100 displays target metabolite b7 in a different manner from the other metabolites (candidate metabolites). In the example in Fig. 21, information processing device 100 displays a block corresponding to target metabolite b7 by surrounding it with a thick line.

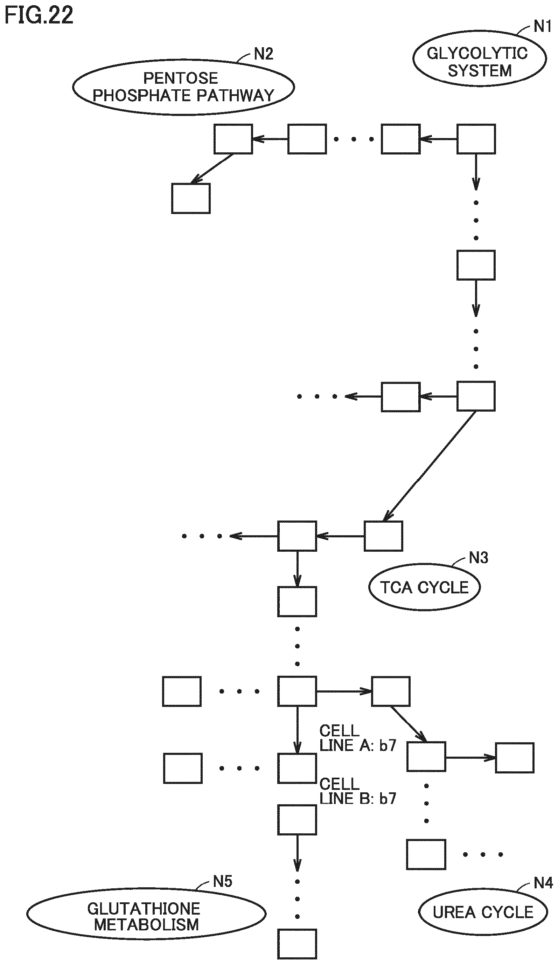

Fig. 22 is a diagram illustrating an example of the metabolic map in this embodiment. In the example in Fig. 22, metabolite b7 of cell line A and metabolite b7 of cell line B are displayed. Also, names of the other metabolites are omitted. Additionally, related information related to the metabolites is displayed below the names of the metabolites. The related information may include a graph or the like indicating a culture parameter (for example, a specific growth speed) of the metabolite in the culture period (10 days), for example. Also, the related information may include a graph indicating a time-series change in amount of metabolite. Moreover, the manufacturer may switch the related information (for example, the specific growth speed) to other related information (for example, cell viability) by executing a predetermined switching operation on input device 40.

Also, the manufacturer can estimate the culture condition to be adjusted in culture device 2 by viewing the fifth image in Fig. 21 and the metabolic map in Fig. 22. For example, the manufacturer can estimate the culture condition to be adjusted by viewing each metabolite or the like included in the metabolic map around target metabolite b7 from the entire metabolic map. Furthermore, the manufacturer can also estimate how much the culture condition to be adjusted is to be adjusted by viewing the metabolic map in the surroundings.

Next, other display images that display device 30 displays will be described. For example, once the manufacturer designates a block of a name of a metabolite from among names of metabolites b 1 to b 12 (including target metabolite b7) displayed in the fifth image in Fig. 21, an image (sixth image) of only the metabolic map around the metabolite, and the metabolic map at another location may not be displayed.

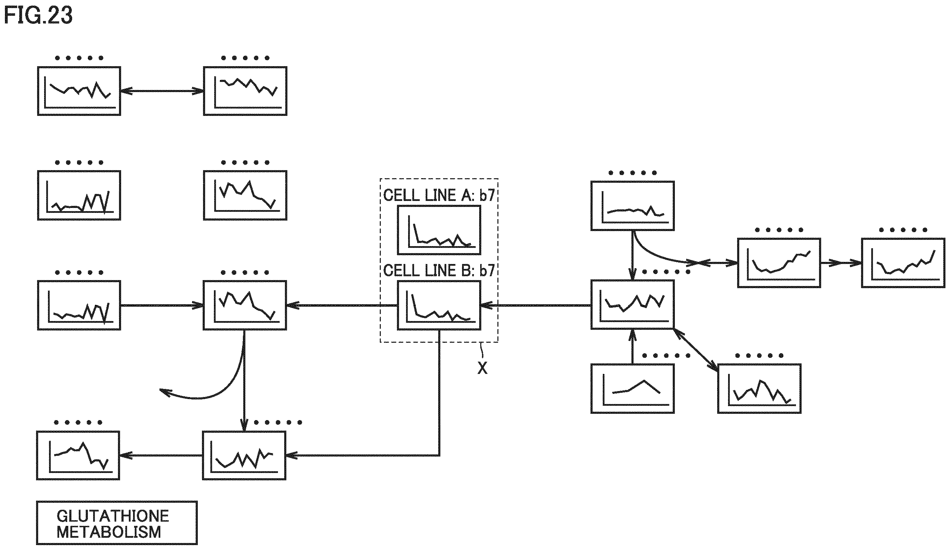

Fig. 23 is an example of an image (sixth image) of the metabolic map around the designated metabolite. The example in Fig. 23 is the metabolic map displayed in a case where target metabolite b7 is designated from among the plurality of metabolites in Fig. 21. In this example, target metabolite b7 is a glutathione metabolic pathway, and a metabolic map corresponding to the glutathione metabolic pathway is thus displayed in the metabolic map. In this manner, only the metabolic map around the designated metabolite is displayed from among the plurality of metabolites that are attributable to the above differences, and the metabolic map at another location is not displayed in the example in Fig. 23. Furthermore, related information of target metabolite b7 is displayed for both cell line A and cell line B in the example in Fig. 23. The related information is a graph illustrating a culture parameter (for example, a specific growth speed) of the metabolite in the culture period (10 days), for example. Also, the manufacturer may switch the related information (specific growth speed) to other related information (cell viability) by executing a predetermined switching operation on input device 40.

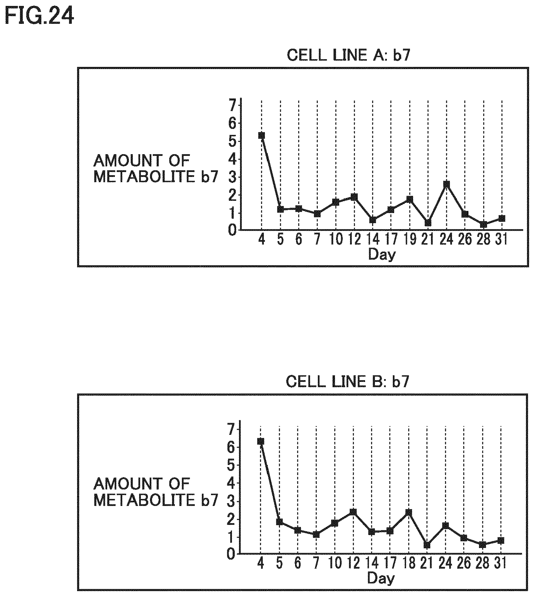

Fig. 24 is an enlarged view of an image X of cell line A and cell line B in Fig. 23. Note that information processing device 100 can cause the screen in Fig. 24 to be displayed by the manufacturer performing a predetermined enlarging operation on the screen in Fig. 23. In the example in Fig. 24, time-series changes in amount of metabolite b7 of cell line A and amount of metabolite b7 of cell line B are displayed. Also, the manufacturer may be able to cause other information (for example, a culture parameter) to be displayed as displayed information by performing a predetermined operation. With such a configuration, the manufacturer can estimate the culture condition to be adjusted in culture device 2 on the basis of the amounts of time-series changes in target metabolite of cell line A and in target metabolite of the cell line B, a culture parameter, and other locations in the metabolic map.

### [Flowchart of information processing device]

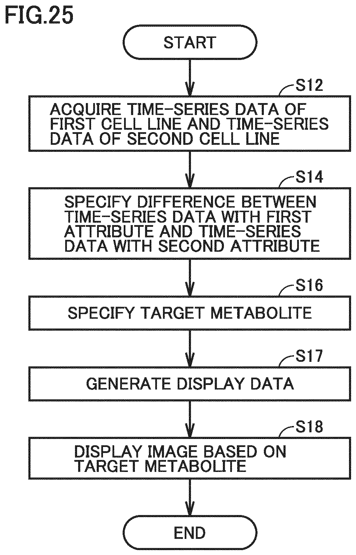

Fig. 25 is a flowchart illustrating main processing of information processing device 100 according to this embodiment. In the example in Fig. 25, information processing device 100 acquires first metabolite data and second metabolite data in Step S12 first. Next, information processing device 100 outputs a request signal, the first metabolite data, and the second metabolite data to computing device 200 in Step S14. Computing device 200 executes principal component analysis on the first metabolite data and the second metabolite data. Computing device 200 generates focused metabolite data through the principal component analysis. Computing device 200 transmits the focused metabolite data to information processing device 100, and information processing device 100 thereby acquires the focused metabolite data. Then, information processing device 100 specifies a difference between the first metabolite data and the second metabolite data on the basis of the focused metabolite data (see Figs. 19 and 20). Next, information processing device 100 specifies target metabolite b7 in Step S16 (see Fig. 21). Next, information processing device 100 generates display data based on target metabolite b7 in Step S17. Then, information processing device 100 displays an image (the above fifth image or sixth image) based on target metabolite b7 on display device 30 in Step S18.

### [Other display images]

Next, other display images of images to be displayed by display device 30 will be described. For example, the configuration in which the image of only the metabolic map around target metabolite b7 that has been designated in the fifth image in Fig. 21 is displayed has been described in Figs. 23 and 24.

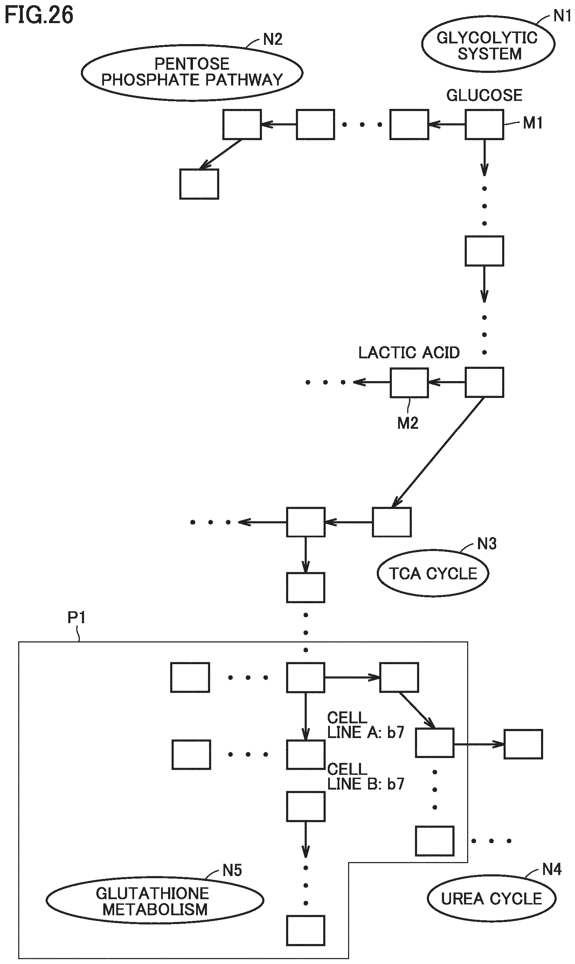

However, information processing device 100 may display the metabolic map displayed in a case where designation has been made from among the plurality of metabolites as an image (seventh image) in a different manner from the metabolic map at the other location. Fig. 26 is a diagram illustrating an example of the seventh image. In the example in Fig. 26, a glutathione metabolic pathway of target metabolite b7 is displayed by being surrounded by a frame P1. In other words, the metabolic map of the glutathione metabolic pathway is displayed in a different manner from the metabolic map at another location. With such a configuration, the manufacturer can more clearly identify the metabolic map around the designated metabolite (target metabolite). Therefore, the manufacturer can more clearly presume the culture condition to be adjusted on the basis of the metabolic map around the designated metabolite. Note that the different manner is not limited to that in the example in Fig. 26 and another manner may be used. For example, blinking or the like of the metabolic map to be displayed in a case where designation has been made from among the plurality of metabolites may be performed.

Also, information processing device 100 may display an eighth image (see Fig. 14) including the name of the metabolic pathway of the designated metabolite. Even with such a configuration, the manufacturer can more clearly presume the culture condition to be adjusted on the basis of the metabolic pathway.

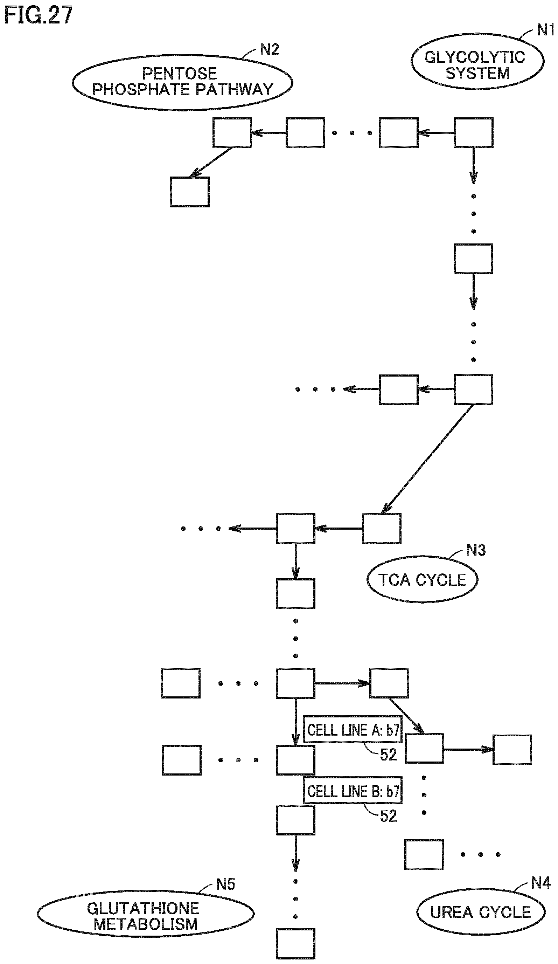

Also, information processing device 100 may display the eighth image such that the designated metabolite is identifiable in the metabolic map. Fig. 27 is an example of the eighth image. As illustrated in Fig. 27, information processing device 100 displays the metabolic map. Along with this, information processing device 100 displays the designated metabolite in a different manner from the other metabolites in the metabolic map. In the example in Fig. 27, it is assumed that target metabolite b7 has been designated by the manufacturer. In this case, information processing device 100 displays designated target metabolite b7 in a different manner from the other metabolites. In the example in Fig. 27, the name of target metabolite b7 is displayed in a manner in which it is surrounded by frame 52. With this configuration, the manufacturer can recognize the metabolite that is attributable to the aforementioned difference. Therefore, the manufacturer can more clearly presume the culture condition to be adjusted on the basis of the metabolite.

Also, the case where there are multiple (twelve in the example in Fig. 21) metabolites that are attributable to the aforementioned difference has been described in this embodiment. However, in a case where there is one metabolite that is attributable to the aforementioned difference, any image out of the seventh image and the eighth image may be displayed for the one metabolite. Also, display of any of the aforementioned first image to the eighth image may be switched by an operation of the manufacturer.

Also, the configuration in which the cell line with the first attribute is cell line A and the cell line with the second attribute is cell line B has been described in this embodiment. However, the cell line with the first attribute and the cell line with the second attribute may be other fine part lines. For example, the cell line with the first attribute may be a cell line cultured under a first culture condition, and the cell line with the second attribute may be a cell line cultured under a second culture condition (which is a culture condition that is different from the first culture condition). With such a configuration, the manufacturer can presume which of the first culture condition and the second culture condition is an appropriate culture condition or the like from a displayed specific cell line. Also, the number of attributes may be three or more.

### [Modification examples]

(1) In the aforementioned embodiment, the configuration in which information processing device 100 generates the display data and causes display device 30 to display the image based on the display data has been described. However, a configuration in which information processing device 100 does not cause display device 30 to display the image may be employed. In a case of such a configuration, the manufacturer causes a recording medium that can record information thereon to be connected to information processing device 100, for example. Then, information processing device 100 causes the recording medium to store the display data. The manufacturer may connect the recording medium storing the display data thereon to a different display device and cause the different display device to display the image based on the display data. Even with such a configuration, effects that are similar to those of the aforementioned embodiment are achieved.
(2) In the aforementioned embodiment, the configuration in which analysis device 6 and information processing device 100 are separate devices has been described. However, analysis device 6 may execute at least a part of processing executed by information processing device 100. For example, analysis device 6 may display the image that display device 30 is caused to display as described above.
(3) In the aforementioned embodiment, the example in which single information processing device 100 executes all the processes has been described. However, one or multiple processes may be executed by another information processing device. The plurality of information processing device may be disposed at remote locations from each other. For example, a certain computer may execute the acquisition of the degrees of relevance, and another computer disposed at a remote location may execute the generation of the display data. A plurality of other computers may be present and perform generation of mutually different display data.
(4) In the aforementioned embodiment, the example in which the metabolite data obtained from the plurality of cell lines are compared and multivariable analysis (principal component analysis) is used to determine the target metabolite has been described. However, correlation analysis may be performed on time-series metabolite data obtained from a single cell line to determine a necessary time range, multivariable analysis may be performed on metabolite data in the time range, and a relationship between the culture parameter and the metabolite data may thus be obtained.

### [Aspects]

Those skilled in the art will understand that the aforementioned plurality of illustrative embodiments are specific examples of the following aspects.

### (Clause 1)

An antibody production assisting method according to an aspect is an antibody production assisting method for assisting production of an antibody, the method including: acquiring time-series data that indicates, in in chronological order, a plurality of metabolite data and a culture parameter, the plurality of metabolite data each corresponding to a respective amount of a plurality of metabolites generated by culturing a cell line for producing the antibody, the culture parameter being detected from the cell line in culture; acquiring a degree of relevance between the culture parameter and each of the plurality of metabolites on the basis of the time-series data; and generating display data based on the degree of relevance.

With such a configuration, the display data based on the degree of relevance is generated. Therefore, the manufacturer can presume a culture condition to be adjusted by viewing an image based on the display data, and as a result, it is possible to assist production of the antibody.

### (Clause 2)

The antibody production assisting method described in the first clause further includes: causing a display device to display an image based on the display data.

With such a configuration, an image related to the display data based on the degree of relevance is displayed. Therefore, the manufacturer can presume a culture condition to be adjusted by viewing the image.

### (Clause 3)

In the antibody production assisting method described in the second clause, the acquiring of the time-series data includes acquiring a plurality of first metabolite data and a plurality of second metabolite data, each of the plurality of first metabolite data corresponding to a respective amount of a plurality of metabolites generated by culturing a cell line with a first attribute, each of the plurality of second metabolite data corresponding to a respective amount of a plurality of metabolites generated by culturing a cell line with a second attribute, the antibody production assisting method further includes: specifying a difference between the first metabolite data and the second metabolite data; and specifying a target metabolite caused by the difference, and the causing of the display device to display includes displaying an image based on the target metabolite.

With such a configuration, the manufacturer can recognize the target metabolite that is caused by the difference between the first metabolite data and the second metabolite data.

### (Clause 4)

In the antibody production assisting method described in the second clause or the third clause, the causing of the display device to display includes displaying, for the culture parameter, a correspondence image in which each of names of the plurality of metabolites is associated with a degree of relevance between the culture parameter and each of the plurality of metabolites.

With such a configuration, the manufacturer can presume a culture condition to be adjusted by viewing the correspondence image.

### (Clause 5)

In the antibody production assisting method described in the fourth clause, the antibody production assisting method further includes: extracting a degree of relevance that satisfies a condition for a degree of relevance indicating that a degree of relevance with the culture parameter is high from among all calculated degrees of relevance, and the correspondence image is an image in which the degree of relevance that satisfies the condition for a degree of relevance is associated with a name of a metabolite associated with the degree of relevance.

With such a configuration, the metabolite with a high degree of relevance with the culture parameter is displayed, and the manufacturer can thus presume a culture condition to be adjusted on the basis of the metabolite.

### (Clause 6)

In the antibody production assisting method described in the fifth clause, the causing of the display device to display includes causing the display device to display the correspondence image in a different manner for each metabolic pathway of the metabolite for the metabolite corresponding to the degree of relevance that satisfies the condition for a degree of relevance.

With such a configuration, the metabolite with a high degree of relevance with the culture parameter is displayed, and the manufacturer can thus presume a culture condition to be adjusted on the basis of the metabolite.

### (Clause 7)

In the antibody production assisting method described in the fifth clause or the sixth clause, the causing of the display device to display includes displaying a metabolic map around the metabolite corresponding to the degree of relevance that satisfies the condition for a degree of relevance and a metabolic map at another location in different manners from each other.

With such a configuration, the manufacturer can recognize the metabolic map around the metabolite with a high degree of relevance with the culture parameter. Therefore, the manufacturer can more clearly presume a culture condition to be adjusted on the basis of the metabolic map.

### (clause 8)

In the antibody production assisting method described in the fifth clause or the sixth clause, the causing of the display device to display includes displaying a metabolic map around the metabolite corresponding to the degree of relevance that satisfies the condition for a degree of relevance and not displaying a metabolic map at another location.

With such a configuration, the manufacturer can recognize the metabolic map around the metabolite with a high degree of relevance with the culture parameter. Therefore, the manufacturer can more clearly presume a culture condition to be adjusted on the basis of the metabolic map.

### (Clause 9)

In the antibody production assisting method described in any one of the fifth clause to the eighth clause, the causing of the display device to display includes displaying a name of a metabolic pathway of the metabolite corresponding to the degree of relevance that satisfies the condition for a degree of relevance.

With such a configuration, the manufacturer can recognize the metabolic pathway of the metabolite with a high degree of relevance with the culture parameter. Therefore, the manufacturer can more clearly presume a culture condition to be adjusted on the basis of the metabolic pathway.

### (Clause 10)

In the antibody production assisting method described in any one of the fourth clause to the ninth clause, the causing of the display device to display includes displaying the metabolite corresponding to the degree of relevance that satisfies the condition for a degree of relevance in an identifiable manner in a metabolic map.

With such a configuration, the manufacturer can recognize the metabolite with a high degree of relevance with the culture parameter. Therefore, the manufacturer can more clearly presume a culture condition to be adjusted on the basis of the metabolite.

### (Clause 11)

An antibody production assisting program according to an aspect is a program for assisting production of an antibody, the program causing a computer to execute: acquiring of time-series data that indicates, in in chronological order, a plurality of metabolite data and a culture parameter, the plurality of metabolite data each corresponding to a respective amount of a plurality of metabolites generated by culturing a cell line for producing the antibody, the culture parameter being detected from the cell line in culture; acquiring of a degree of relevance between the culture parameter and each of the plurality of metabolites on the basis of the time-series data; and generating of display data based on the degree of relevance.

With such a configuration, the display data based on the degree of relevance is generated. Therefore, the manufacturer can presume a culture condition to be adjusted by viewing an image based on the display data, and as a result, it is possible to assist production of the antibody.

The embodiments disclosed herein should be considered as being illustrative examples and not limitations in any sense. The scope of the present disclosure is indicated by the scope of the claims rather than the above description of the embodiments, and is intended to include all modifications within meanings and a scope equivalent to the scope of the claims.

### REFERENCE SIGNS LIST

1 assisting system; 2 culture device; 3 culture condition; 4 sensor group; 6 analysis device; 10 first culture device; 30 display device; 30A display region; 40 input device; 48 name image; 50 network; 100 information processing device; 102 input unit; 104 request unit; 106 display control unit; 162 ROM; 164 RAM; 200 computing device

## Claims

1. An antibody production assisting method for assisting production of an antibody, the method comprising:
acquiring time-series data that indicates, in chronological order, a plurality of metabolite data and a culture parameter, the plurality of metabolite data each corresponding to a respective amount of a plurality of metabolites generated by culturing a cell line for producing the antibody, the culture parameter being detected from the cell line in culture;
acquiring a degree of relevance between the culture parameter and each of the plurality of metabolites on the basis of the time-series data; and
generating display data based on the degree of relevance.

2. The antibody production assisting method according to claim 1, further comprising: causing a display device to display an image based on the display data.

3. The antibody production assisting method according to claim 2,
wherein the acquiring of the time-series data includes acquiring a plurality of first metabolite data and a plurality of second metabolite data, each of the plurality of first metabolite data corresponding to a respective amount of a plurality of metabolites generated by culturing a cell line with a first attribute, each of the plurality of second metabolite data corresponding to a respective amount of a plurality of metabolites generated by culturing a cell line with a second attribute,
the antibody production assisting method further comprises:
specifying a difference between the first metabolite data and the second metabolite data; and
specifying a target metabolite caused by the difference, and
the causing of the display device to display includes displaying an image based on the target metabolite.

4. The antibody production assisting method according to claim 2, wherein the causing of the display device to display includes displaying, for the culture parameter, a correspondence image in which each of names of the plurality of metabolites is associated with a degree of relevance between the culture parameter and each of the plurality of metabolites.

5. The antibody production assisting method according to claim 4,
further comprising extracting a degree of relevance that satisfies a condition for a degree of relevance indicating that a degree of relevance with the culture parameter is high from among all calculated degrees of relevance, wherein
the correspondence image is an image in which the degree of relevance that satisfies the condition for a degree of relevance is associated with a name of a metabolite associated with the degree of relevance.

6. The antibody production assisting method according to claim 5, wherein the causing of the display device to display includes causing the display device to display the correspondence image in a different manner for each metabolic pathway of the metabolite for the metabolite corresponding to the degree of relevance that satisfies the condition for a degree of relevance.

7. The antibody production assisting method according to claim 5, wherein the causing of the display device to display includes displaying a metabolic map around the metabolite corresponding to the degree of relevance that satisfies the condition for a degree of relevance and a metabolic map at another location in different manners from each other.

8. The antibody production assisting method according to claim 5, wherein the causing of the display device to display includes displaying a metabolic map around the metabolite corresponding to the degree of relevance that satisfies the condition for a degree of relevance and not displaying a metabolic map at another location.

9. The antibody production assisting method according to claim 5, wherein the causing of the display device to display includes displaying a name of a metabolic pathway of the metabolite corresponding to the degree of relevance that satisfies the condition for a degree of relevance.

10. The antibody production assisting method according to claim 5, wherein the causing of the display device to display includes displaying the metabolite corresponding to the degree of relevance that satisfies the condition for a degree of relevance in an identifiable manner in a metabolic map.

11. A program for assisting production of an antibody,
the program causing a computer to execute:
acquiring of time-series data that indicates, in in chronological order, a plurality of metabolite data and a culture parameter, the plurality of metabolite data each corresponding to a respective amount of a plurality of metabolites generated by culturing a cell line for producing the antibody, the culture parameter being detected from the cell line in culture;
acquiring of a degree of relevance between the culture parameter and each of the plurality of metabolites on the basis of the time-series data; and
generating of display data based on the degree of relevance.
